# EUROPEAN PATENT SPECIFICATION

(11) **EP 0 495 441 B1**
(45) Date of publication and mention of the grant of the patent: **13.09.1995**
(21) Application number: 92100470.1
(22) Date of filing: 13.01.1992
(51) Int. Cl.: A23K 1/00

(54) **Feed additive for ruminants**
Futterzusätze für Wiederkäuer
Additif alimentaire destiné aux ruminants

(30) Priority: 14.01.1991 JP 70265/91; 18.12.1991 JP 335073/91
(43) Date of publication of application: 22.07.1992
(73) Proprietor: Ajinomoto Co., Inc., Tokyo 104 (JP)
(72) Inventor: Ueda, Satoshi, c/o Central Research Laboratories, Kawasaki-ku, Kawasaki-shi, Kanagawa-ken (JP); Iizuka, Takashi, c/o Central Research Laboratories, Kawasaki-ku, Kawasaki-shi, Kanagawa-ken (JP); Heima, Haruo, c/o Central Research Laboratories, Kawasaki-ku, Kawasaki-shi, Kanagawa-ken (JP); Ozawa, Makoto, c/o Central Research Laboratories, Kawasaki-ku, Kawasaki-shi, Kanagawa-ken (JP); Nagai, Takeshi, c/o Central Research Laboratories, Kawasaki-ku, Kawasaki-shi, Kanagawa-ken (JP); Sato, Hiroyuki, c/o Central Research Laboratories, Kawasaki-ku, Kawasaki-shi, Kanagawa-ken (JP)
(74) Representative: Strehl Schübel-Hopf Groening & Partner

(56) References cited:
- GB-A- 2 153 199
- US-A- 3 413 118
- CHEMICAL ABSTRACTS, vol. 115, no. 13, 30 September 1991, Columbus, Ohio, US;abstract no. 134668a, page 842 ;column 2 ;
- PATENT ABSTRACTS OF JAPAN vol. 11, no. 18 (C-398)(2465) 17 January 1987PATENT ABSTRACTS OF JAPAN vol. 13, no. 158 (C-586)(3506) 17 April 1989
- JOURNAL OF DAIRY SCIENCE. vol. 73, no. 10, 1990, CHAPAIGN, ILLINOIS US pages2934 - 2939 T. C. JENKINS 'Nutrient digestion, ruminal fermentation, and plasmalipids in steers fed combinations of hydrogenated fat and lecithin'

## Description

### Field of the invention

This invention relates to a feed additive for ruminants. More specifically, it relates to a feed additive composition having a biological active substance coated with a coating composition which is stable in the rumen of the ruminant and can be released in the abomasum or its subsequent digestive tract and makes it possible to digest the biologically active substance in the abomasum and the subsequent digestive tract.

In ruminant animals such as cattle or sheep, the direct oral administration of biologically active substances such as amino acids and vitamins leads to the decomposition of most of them by microorganisms in the rumen, and they are not effectively utilized.

Accordingly, it is important in the field of feeds, nutrient preparations and veterinary drugs for ruminant animals to pass the biologically active substances through the rumen without being decomposed by microorganisms in the rumen so that they can be effectively digested and absorbed in the abomasum and the subsequent digestive tract.

It was proposed in the past to coat ruminant animal feed additives containing biologically active substances with protective substances such as fatty acids, and hardened animal oils and hardened vegetable oils. But the particles coated with these fats and oils are so stable not only in the rumen but also in the abomasum and the subsequent digestive tract, that it is difficult to release the biologically active substances in the abomasum and the subsequent digestive tract.

For this reason, some methods of adding substances to protective substances propelling the release of the biologically active substances in the abomasum and the subsequent digestive tract were proposed. In these methods, the biologically active substances are dispersed in coating materials and granulated or are coated with coating materials.

As a method of dispersing the biologically active substances Japanese Laid-Open Patent Publication No. 168351/85 proposes the method of dispersing a biologically active substance in a protective substance which comprises granulating a biologically active substance containing at least 20% by weight of calcium carbonate and at least 10% by weight of a substance selected from the group consisting of a monocarboxylic acid, a hardened oil and a fat. Furthermore, Japanese Laid-Open Patent Publication No. 195653/86 proposes a process for dispersing the biologically active substances in a coating material composed of at least 10% by weight of a substance selected from the group consisting of a monocarboxylic acid, a hardened oil and a fat and at least 20% by weight to not more than 50% by weight of an insoluble salt of an acid which is less acidic than hydrochloric acid.

As a method of coating with coating materials, for example, Japanese Laid-Open Patent Publication No. 317053/88 proposes the method, which comprises coating a biologically active substance with coating materials containing the protective substance composed of a monocarboxylic acid, a hardened oil, lecithin, and a glycerin fatty acid ester.

However, by the method of dispersing a biologically active substance in a coating material, since the biologically active substance is present near the surface of the particle, the content of the biologically active substance should be considerably decreased in order to attain the protection. In view of the fact that the passage time from rumen to abomasum is several hours to several days, it is difficult to keep the biologically active substance which is present near the surface stable in rumen. Furthermore, when it is coated with a coating material composed of lecithin, a glycerin fatty acid ester and an oil or a fat, the coating layer has insufficient strength and its protective ability leaves a problem. On the other hand, lecithin and a glycerin fatty acid ester are expected to have an emulsifying effect on the oil and fat in the small intestine. But in view of the time which is required to pass through the small intestine, the ability to release the biologically active substances is not sufficient.

To utilize the difference in pH between the rumen and the abomasum, it is proposed to use a method of coating with a polymer which is insoluble in the environment of the rumen, but is soluble in the strongly acidic abomasum. Since an organic solvent is used for coating and the coating agent becomes expensive, this procedure is not fully satisfactory.

### Detailed description of the invention

The problem to be solved by the present invention is to protect a biologically active substance stably in the rumen of a ruminant animal, and to release it in order to be digested and absorbed efficiently in the abomasum and the subsequent digestive tract, by safe and economical means.

The present inventors made extensive efforts to achieve the above object, and found that by coating a core containing a biologically active substance with coating materials containing lecithin, inorganic substances which are stable under neutral conditions but soluble under acidic conditions, and at least one substance selected from the group consisting of straight-chain or branched-chain saturated or unsaturated monocarboxylic acids having 14 to 22 carbon atoms or salts thereof, hardened vegetable oils, hardened animal oils, and waxes, excellent stability in the rumen and excellent dissolution in the abomasum and the subsequent digestive tract can be concurrently provided. This led to the present invention.

The essence of this invention is a feed additive for ruminants comprising a core containing a biologically active substance and a coating composition placed on the surface of said core,
said coating composition comprising lecithin, at least one inorganic substance which is stable in a neutral medium and soluble under acidic conditions, and at least one substance selected from the group consisting of straight-chain or branched-chain saturated or unsaturated monocarboxylic acids having 14 to 22 carbon atoms, salts thereof, hardened vegetable oils, hardened animal oils, and waxes.

The feed additive for ruminants according to this invention contains in the coating material lecithin and inorganic substances which are stable under neutral but soluble under acidic conditions. By utilizing the fact that the inside of the abomasum is acidic, the dissolution of the inorganic substances and the emulsifying effect of lecithin to fatty acids and hardened oils in the small intestine causes the release of the biologically active substances in the abomasum and its subsequent digestive tract, and its synergistic effect results in good dissolving properties. Furthermore, the addition of the inorganic acids or their salts increases the strength of the coating layer.

In the present invention, the biologically active substances are at least one material selected from known nutrients, feeds containing them, drugs, such as amino acids and derivatives thereof, hydroxy homologous compounds of amino acids, proteins, hydrocarbons, vitamins and veterinary medicines.

Specifically, they include amino acids such as lysine, methionine, tryptophan and threonine; amino acid derivatives such as N-acylamino acids and N-hydroxymethylmethionine calcium salt, and lysine hydrochloride; hydroxy homologous compounds of amino acids such as 2-hydroxy-4-methylmercaptobutyric acid and salts thereof; powders of natural nutrients such as grain powders, feathers and fish powder; proteins such as casein, corn proteins and potato proteins; carbohydrates such as starch, cane sugar and glucose; vitamins and substances having a similar function such as vitamin A, vitamin A acetate, vitamin A palmitate, vitamins B, thiamine, thiamine hydrochloride, riboflavin, nicotinic acid, nicotinic acid amide, calcium pantothenate, choline pantothenate, pyridoxine hydrochloride, choline chloride, cyanocobalamine, biotin, folic acid, p-aminobenzoic acid, vitamin D₂, vitamin D₃ and vitamin E; antibiotics such as tetracyclic antibiotics, amino glycoside antibiotics, macrolide-type antibiotics, polyether-type antibiotics, insecticides such as negfon; vermicides such as piperazine, and hormones such as estrogen, stilbestrol, hexestrol, tyroprotein and goitrogen.

There is no particular restriction in the preparative method of the cores containing the biologically active substance. If required, a binder or a filler may be added, and granules, preferably particles having a nearly spherical shape, are prepared by a usual granulating method, such as an extrusion granulating method, a fluidized granulating method, and a stirred granulating method.

Examples of the binder are cellulose derivatives such as hydroxypropylcellulose, methyl cellulose, or sodium carboxymethylcellulose, vinyl derivatives such as polyvinyl alcohol or polyvinylpyrrolidone, gum arabic, guaiac gum and sodium polyacrylate.

Starch, proteins and crystalline cellulose may be used as the filler. If required, a specific gravity adjusting agent may be added such as calcium carbonate, calcium phosphonate and talc.

The coating material for coating a core containing the biologically active substance comprise lecithin, at least one inorganic substance which is stable in the neutral medium, but soluble under acidic conditions and at least one substance selected from the group consisting of straight-chain or branched-chain saturated or unsaturated monocarboxylic acids having 14 to 22 carbon atoms, or salts thereof, hardened vegetable oils, hardened animal oils, and waxes.

Examples of monocarboxylic acids include myristic acid, palmitic acid, stearic acid, oleic acid, linoleic acid, and behenic acid. Salts of these may also be used. Examples of hardened vegetable oils include hardened palm oil, hardened soybean oil, hardened rapeseed oil, and hardened castor oil. Examples of hardened animal oils are hardened beef tallow and hog fat. Examples of waxes are carnauba wax and beeswax, natural waxes, synthetic waxes, and paraffin waxes.

Lecithin used in the invention is not required to be pure. A mixture of phosphatidyl choline, phosphatidyl ethanolamine, phosphatidyl inositol may be used. Preferably, it may be prepared from soybean and egg yolk.

Examples of the inorganic substance which is stable under neutral conditions and soluble under acidic conditions include magnesium carbonate, calcium carbonate, calcium phosphate, calcium pyrophosphate and mixtures thereof. Carbonates such as magnesium carbonate and calcium carbonate and calcium salt of pyrophosphoric acid are more preferred.

The coating materials of this invention comprise 0.1 to 20% by weight of lecithin, 0.1 to 10% by weight of the inorganic substance which is stable in neutrality and soluble under acidic conditions, preferably 1 to 10% of lecithin and 1 to 10% of the inorganic substance. If the amount of lecithin in the coating materials exceeds 20% by weight, the strength of the coating layer is decreased and the protecting ability in the rumen is reduced. If the amount of lecithin is less than 0.1% by weight, the emulsifying effect is insufficient, and the dissolving effect in the abomasum and its subsequent digestive tract decreases. If the amount of the inorganic substance which is stable in neutrality and soluble under acidic conditions exceeds 10% by weight, the protecting ability in the rumen decreases. If it is below 0.1% by weight, the effect of the inorganic substance which utilizes the fact that the inside of the abomasum is acidic, is insufficient.

The ruminant animal feed additive composition of this invention is characterized by the fact that the core containing the biologically active substance is coated with the coating material.

The amount of the coating material which is used is not restricted in particular. It should be as small as possible, because the amount of the biologically active substance is large. But it should be enough to enable the coating material to fully protect the biologically active substance in the rumen. Usually, it is used for coating in an amount of 10 to 200 parts by weight, preferably 15 to 150 parts by weight, per 100 parts by weight of the core containing the biologically active substance.

There is no particular restriction on the method of coating. Any ordinary coating method can be applied, such as the method of fluidized-bed coating, the method of pan coating or the method of melt coating.

The present invention will be illustrated by Examples, and Comparative Examples, but the scope of the invention should not be limited to these examples.

### Examples

The utility of the ruminant animal feed additive was evaluated by the following methods.

### Stability in the rumen

About 2 g of the prepared sample was put in a 200 ml Erlenmeyer flask. 100 ml of Mc Dougall buffer solution which corresponds to the rumen juice was put in the container, and shaken for 48 hours at a temperature of 39^{o}C. After shaking, the amount of the biologically active substance dissolved was analyzed, and the stability in the rumen was calculated.

As the biologically active substance, the amount of amino acid dissolved in the examples was analyzed by liquid chromatography.

### Mc Dougall buffer solution *

The following reagents were dissolved in 1000 ml of water.
Sodium hydrogen carbonate: 7.43 g
Disodium phosphate 12-hydrate: 7.00 g
Sodium chloride: 0.34 g
Potassium chloride: 0.43 g
Magnesium chloride hexahydrate: 0.10 g
Calcium chloride: 0.005 g

### Dissolving property in the abomasum

After the stability test, the shaken sample was recovered and washed, and further put into a 200 ml Erlenmeyer flask. 40 ml of a Clark-Lubs buffer solution corresponding to the abomasum juice was added, and shaken for 3 hours at 39^{o}C. After shaking, the amount of the biologically active substance dissolved was analyzed, and the dissolving property in the abomasum was calculated.

### Clark-Lubs buffer solution *

A buffer solution obtained by dissolving the following reagents in 1000 ml of water.
Potassium chloride: 3.73 g
Hydrochloric acid: 2.1 ml.

### Dissolving property in the small intestine

After the dissolving property test inside the abomasum, the shaken sample was recovered, and further put into a 200 ml Erlenmeyer flask. 100 ml buffer solution corresponding to a small intestine juice was added, and shaken for 24 hours at 39^{o}C. After shaking, the amount of the dissolved biologically active substance was analyzed, and the dissolving property in the small intestine was calculated.

### Example 1

A kneader was charged with 325 g of L-lysine hydrochloride, 172.5 g of talc, 2.5 g of sodium carboxymethylcellulose and 135 g of water. They were kneeded and then granulated by an extruder having a screen with an opening size of 1.5 mm, where cylindrical granules were obtained. The granules obtained were molded by using a spherical granule producing machine (Marumerizer. Fumi Paudal Co., Ltd.) to obtain granules having a nearly spherical shape. The resulting spherical granules were dried in a fluidized-bed dryer to obtain cores containing L-lysine hydrochloride.

A coating material consisting of 5 parts by weight of lecithin (soybean lecithin, manufactured by WAKO Pure Chemical Industries, Ltd. - food additive - was used) and 5 parts by weight of magnesium carbonate dispersed per 90 parts by weight of melted hardened beef tallow was prepared. The coating material was used in an amount of 67 parts by weight per 100 parts of the cores (rate of coating 40%). A fluidized-bed coater (New Marumerizer, Fuji Paudal Co., Ltd.) was used.

The coated particles were subjected to the above evaluation tests and the results were as follows:
The dissolving rate in the rumen was 9%; the dissolving rate in the abomasum was 39%; and the dissolving rate in the small intestine was 40%.

### Example 2

The preparation was conducted in the same manner as in Example 1 except that in Example 2, calcium carbonate was used instead of magnesium carbonate.

The coated particles were subjected to the above evaluation tests, and the results were as follows:
The dissolving rate in the rumen was 4%; the dissolving rate in the abomasum was 46%; and the dissolving rate in the small intestine was 36%.

### Example 3

The preparation was conducted in the same manner as in Example 2 except that in Example 3 the coating material was used in an amount of 43 parts by weight per 100 parts of the cores (rate of coating 30%).

The coated particles were subjected to the above evaluation tests, and the results were as follows:
The dissolving rate in the rumen was 12%; the dissolving rate in the abomasum was 20%; and the dissolving rate in the small intestine was 58%.

### Example 4

The preparation was conducted in the same manner as in Example 2 except that in Example 4 the coating material contains 10 parts by weight of lecithin and 5 parts by weight of calcium carbonate and was used in an amount of 33 parts by weight (rate of coating 25%).
The coated particles were subjected to the above evaluation tests, and the results were as follows:
The dissolving rate in the rumen was 19%; the dissolving rate in the abomasum was 46%; and the dissolving rate in the small intestine was 32%.

### Example 5

The preparation was conducted in the same manner as in Example 2 except that in Example 5 the coating material contains 2 parts by weight of lecithin and 10 parts by weight of calcium carbonate and was used in an amount of 25 parts by weight (rate of coating 20%).

The coated particles were subjected to the above evaluation tests, and the results were as follows:
The dissolving rate in the rumen was 14%; the dissolving rate in the abomasum was 31%; and the dissolving rate in the small intestine was 35%.

### Example 6

The preparation was conducted in the same manner as in Example 3 except that in example 6 calcium pyrophosphate was used instead of calcium carbonate.
The coated particles were subjected to the above evaluation tests, and the results were as follows:
The dissolving rate in the rumen was 7%; the dissolving rate in the abomasum was 49%; and the dissolving rate in the small intestine was 32%.

### Example 7

A kneader was charged with 375 g of D.L-methionine, 120 g of talc, 5 g of sodium carboxylmethylcellulose and 150 g of water, which were kneeded. The mixture was processed by using an extruder having a screen with an opening size of 1.5 mm to obtain cylindrical granules. The resulting granules were formed by a spherical shaping apparatus (Marumerizer, Fuji Paudal Co., Ltd.) to obtain granules having a nearly spherical shape. The spherical granules were dried by a fluidized-bed dryer to obtain cores containing D.L-methionine.

A coating material consisting of 5 parts by weight of lecithin and 5 parts by weight of magnesium carbonate dispersed per 90 parts by weight of melted hardened beef tallow was prepared. The coating material was used in an amount of 43 parts by weight per 100 parts by weight of the cores (rate of coating 30%). A fluidized-bed coater (New Marumerizer) was used.

The coated particles were subjected to the above evaluation tests and the results were as follows:
The dissolving rate in the rumen was 17%; the dissolving rate in the abomasum was 20%; and the dissolving rate in the small intestine was 60%.

### Example 8

The preparation was conducted in the same manner as in Example 7 except that in Example 8 calcium carbonate was used instead of magnesium carbonate.

The coated particles were subjected to the above evaluation tests and the results were as follows:
The dissolving rate in the rumen was 15%; the dissolving rate in the abomasum was 26%; and the dissolving rate in the small intestine was 59%.

### Example 9

The preparation was conducted in the same manner as in Example 8 except that in Example 9 the coating material contains 10 parts by weight of lecithin and 2 parts by weight of calcium carbonate and was used in an amount of 33 parts by weight (rate of coating 25%).

The coated particles were subjected to the above evaluation tests, and the results were as follows:
The dissolving rate in the rumen was 21%; the dissolving rate in the abomasum was 38%; and the dissolving rate in the small intestine was 37%.

### Comparative Example 1

The preparation was conducted in the same manner as in Example 1 except that in comparative Example 1 the coating material contains 10 parts by weight of calcium carbonate.

The coated particles were subjected to the above evaluation tests, and the results were as follows:
The dissolving rate in the rumen was 5%; the dissolving rate in the abomasum was 15%; and the dissolving rate in the small intestine was 16%.

### Comparative Example 2

The preparation was conducted in the same manner as in Example 3 except that in comparative example 2 the coating material contains 30 parts by weight of lecithin. The coated particles were subjected to the above evaluation tests and the results were as follows:
The dissolving rate in the rumen was 71%; the dissolving rate in the abomasum was 27%; and the dissolving rate in the small intestine was 1%.

### Comparative Example 3

The preparation was conducted in the same manner as in Example 8 except that in Comparative Example 3 the coating material contains 30 parts by weight of calcium carbonate.

The coated particles were subjected to the above evaluation tests, and the results were as follows:
The dissolving rate in the rumen was 82%; the dissolving rate in the abomasum was 10%; and the dissolving rate in the small intestine was 3%.

The obtained results are summarized in Tables 1 and 2.

**Table 1**

| Example | | 1 | 2 | 3 | 4 | 5 | 6 |
|---|---|---|---|---|---|---|---|
| Biologically active substance | | L-Lysin hydrochloride | L-Lysin hydrochloride | L-Lysin hydrochloride | L-Lysin hydrochloride | L-Lysin hydrochloride | L-Lysin hydrochloride |
| Cores (parts by weight) | | 100 | 100 | 100 | 100 | 100 | 100 |
| Coated layer (parts by weight) | | 67 | 67 | 43 | 33 | 25 | 43 |
| Composition of the coating layer (%) | Beef tallow | 90 | 90 | 90 | 85 | 88 | 90 |
| | Lecithin | 5 | 5 | 5 | 10 | 2 | 5 |
| | Magnesium carbonate | 5 | - | - | - | - | - |
| | Calcium carbonate | - | 5 | 5 | 5 | 10 | - |
| | Calcium pyrophosphate | - | - | - | - | - | 5 |
| Dissolving rate (%) | Corresponding to the rumen | 9 | 4 | 12 | 19 | 14 | 7 |
| | Corresponding to the abomasum | 39 | 46 | 20 | 46 | 31 | 49 |
| | Corresponding to the small intestine | 40 | 36 | 58 | 32 | 35 | 32 |

**Table 2**

| Example | | 7 | 8 | 9 | | | |
|---|---|---|---|---|---|---|---|
| Comparative Example | | | | | 1 | 2 | 3 |
| Biologically active substance | | D.L-Methionin | D.L-Methionin | D.L-Methionin | Lysin hydrochloride | Lysin hydrochloride | D.L-Methionin |
| Cores (parts by weight) | | 100 | 100 | 100 | 100 | 100 | 100 |
| Coated layer (parts by weight) | | 43 | 43 | 33 | 67 | 43 | 43 |
| Composition of the coating layer (%) | Beef tallow | 90 | 90 | 88 | 90 | 70 | 70 |
| | Lecithin | 5 | 5 | 10 | - | 30 | - |
| | Magnesium carbonate | 5 | - | - | - | - | - |
| | Calcium carbonate | - | 5 | 2 | 10 | - | 30 |
| Dissolving rate (%) | Corresponding to the rumen | 17 | 15 | 21 | 5 | 71 | 82 |
| | Corresponding to the abomasum | 20 | 26 | 38 | 15 | 27 | 10 |
| | Corresponding to the small intestine | 60 | 59 | 37 | 16 | 1 | 3 |

### Effect of the Invention

By coating a core containing a biologically active substance with a coating composition containing lecithin, at least one inorganic substance which is stable under neutral conditions and soluble under acidic conditions, and at least one substance selected from the group consisting of straight-chain of branched chain saturated or unsaturated monocarboxylic acids having 14 to 22 carbon atoms, salts thereof, hardened vegetable oils, hardened animal oils, and waxes, a feed additive for ruminants is obtained which has a better effect than the prior art regarding the protection in the rumen and dissolving properties in the abomasum and the subsequent digestive tract.

The present invention provides a feed composition which enables a biologically active substance to be effectively absorbed by ruminant animals, which has a great importance in industry.

## Claims

1. A feed additive for ruminants comprising a core containing a biologically active substance and a coating composition placed on the surface of said core, said coating composition comprising lecithin, at least one inorganic substance which is stable under neutral conditions and soluble under acidic conditions, and at least one substance selected from the group consisting of straight-chain or branched-chain saturated or unsaturated monocarboxylic acids having 14 to 22 carbon atoms, salts thereof, hardened vegetable oils, hardened animal oils, and waxes.

2. A feed additive for ruminants according to claim 1, wherein said lecithin is used in an amount of from 0.1 to 20% by weight and said inorganic substance stable under neutral conditions and soluble under acidic conditions is used in an amount of from 0.1 to 10% by weight, based on the weight of said coating composition.

3. A feed additive for ruminants according to claim 1 or 2, wherein said inorganic substance which is stable under neutral conditions and soluble under acidic conditions is a carbonate.

4. A feed additive for ruminants according to claim 1 or 2, wherein said inorganic substance which is stable under neutral conditions and soluble under acidic conditions is a calcium salt of pyrophosphoric acid.

5. Feed for ruminants comprising a feed additive according to any one of the claims 1 to 4.

## Patentansprüche

1. Futterzusatz für Wiederkäuer, welcher einen eine biologisch aktive Substanz enthaltenden Kern und eine auf die Oberfläche dieses Kerns aufgebrachte Überzugs-Zusammensetzung aufweist, wobei die Überzugs-Zusammensetzung Lecithin, mindestens eine unter neutralen Bedingungen stabile und unter sauren Bedingungen lösliche anorganische Substanz und mindestens eine Substanz enthält, die aus der aus geradekettigen oder verzweigt-kettigen gesättigten oder ungesättigten Monocarbonsäuren mit 14 bis 22 Kohlenstoffatomen, deren Salzen, gehärteten Pflanzenölen, gehärteten tierischen Ölen und Wachsen bestehenden Gruppe ausgewählt ist.

2. Futterzusatz für Wiederkäuer nach Anspruch 1, wobei das Lecithin in einer Menge von 0,1 bis 20 Gew.-% und die unter neutralen Bedingungen stabile und unter sauren Bedingungen lösliche anorganische Substanz in einer Menge von 0,1 bis 10 Gew.-% bezogen auf das Gewicht der Überzugs-Zusammensetzung, angewendet werden.

3. Futterzusatz für Wiederkäuer nach Anspruch 1 oder 2, wobei die unter neutralen Bedingungen stabile und unter sauren Bedingungen lösliche anorganische Substanz ein Carbonat ist.

4. Futterzusatz für Wiederkäuer nach Anspruch 1 oder 2, wobei die unter neutralen Bedingungen stabile und unter sauren Bedingungen lösliche anorganische Substanz ein Calziumsalz der Pyrophosphorsäure ist.

5. Futter für Wiederkäuer, enthaltend einen Futterzusatz nach einem der Ansprüche 1 bis 4.

## Revendications

1. Additif alimentaire pour ruminants comprenant un coeur contenant une substance biologiquement active et une composition de revêtement disposée sur la surface dudit coeur, ladite composition de revêtement comprenant de la lécithine, au moins une substance inorganique qui est stable dans des conditions neutres et qui est soluble dans des conditions acides, ainsi qu'au moins une substance choisie dans le groupe constitué d'acides monocarboxyliques à chaîne linéaire ou à chaîne ramifiée, saturés ou insaturés, en C₁₄-C₂₂, de leurs sels, d'huiles végétales durcies, d'huiles animales durcies et de cires.

2. Additif alimentaire pour ruminants selon la revendication 1, dans lequel on utilise ladite lécithine à raison de 0,1 à 20% en masse et on utilise ladite substance inorganique qui est stable dans des conditions neutres et qui est soluble dans des conditions acides à raison de 0,1 à 10% en masse par rapport à la masse de ladite composition de revêtement.

3. Additif aimentaire pour ruminants selon la revendication 1 ou 2, dans lequel ladite substance inorganique qui est stable dans des conditions neutres et qui est soluble dans des conditions acides est un carbonate.

4. Additif alimentaire pour ruminants selon la revendication 1 ou 2, dans lequel ladite substance inorganique qui est stable dans des conditions neutres et qui est soluble dans des conditions acides est un sel calcique d'acide pyrophosphorique.

5. Aliment pour ruminants comprenant un additif alimentaire selon l'une quelconque des revendications 1 à 4.
